# EUROPEAN PATENT SPECIFICATION

(11) **EP 2 072 062 B1**
(45) Date of publication and mention of the grant of the patent: **06.06.2012**
(21) Application number: 07025065.9
(22) Date of filing: 22.12.2007
(51) Int. Cl.: A61K 49/10, C07D 401/14

(54) **The use of the spin crossover complex [M*+ (L2) Hw]AN)2 as a magnetic resonance imaging contrast agent**
Verwendung des Spin-Crossover-Komplexes [M*+ (L2) Hw]AN)2 als Kontrastmittel zur Bildgebung durch magnetische Resonanz
Utilisation du complexe de transition de spin [M*+ (L2) Hw]AN)2 en tant qu'agent de contraste d'imagerie par résonance magnétique

(43) Date of publication of application: 24.06.2009
(73) Proprietor: Karlsruher Institut für Technologie, 76131 Karlsruhe (DE)
(72) Inventor: Rajadurai, Chandrasekar Dr., Athikulam Madurai-625007 (IN); Ruben, Mario, 6700 Strasbourg (FR); Kruk, Danuta Prof. Dr., 30150 Krakow (PL)

(56) References cited:
- MULLER ROBERT N. ET AL.: "Spin transition molecular materials: Intelligent contrast agents for magnetic resonance imaging" JOURNAL OF THE AMERICAN CHEMICAL SOCIETY, vol. 125, no. 27, 9 July 2003 (2003-07-09), pages 8405-8407, XP002478064
- RAJADURAI CHANDRASEKAR ET AL: "Spin Transition in a Chainlike Supramolecular Iron(II) Complex" INORGANIC CHEMISTRY, AMERICAN CHEMICAL SOCIETY. EASTON, US, vol. 45, no. 25, 2006, pages 10019-10021, XP002472201 ISSN: 0020-1669
- RAJADURAI C. ET AL.: "Above room temperature spin transition in a metallo-supramolecular coordination oligomer/polymer" CHEMICAL COMMUNICATIONS, vol. 25, May 2007 (2007-05), pages 2636-2638, XP002478130
- THOMPSON ET AL: "Structural studies of thermal- and light- induced transitions in iron(II) spin-crossover complexes" COMPTES RENDUS - CHIMIE, ELSEVIER, PARIS, FR, vol. 8, no. 9-10, September 2005 (2005-09), pages 1365-1373, XP005096099 ISSN: 1631-0748
- Ivan Salitros ET AL: "Room-temperature spin-transition iron compounds", Monatshefte für Chemie - Chemical Monthly, vol. 140, no. 7, 25 March 2009 (2009-03-25), pages 695-733, XP55005997, ISSN: 0026-9247, DOI: 10.1007/s00706-009-0128-4

## Description

The invention relates to the use of the spin crossover complex [M^{x+}(Ly)H_{w}]AN)_{z} as a contrast agent in magnetic resonance imaging (MRI) according to claim 1.

A very active field of application of nuclear relaxation enhancement is the development and use of magnetic materials as contrast agents in magnetic resonance imaging. The magnetic species enhance the proton relaxation rates due to a random variation of the electron spin - nuclear spin interactions (the dipole-dipole interaction and the magnetic hyperfine interaction between the nuclear and electron magnetic moments), which open new pathways for longitudinal as well as transverse relaxation.

A well known category of contrast agents is paramagnetic solutions of transition metal complexes (very popular are Gd and Mn based contrast agents). In this case the origin of the nuclear relaxation enhancement is found in the value of the electronic magnetic moment (about 650 times that of the proton). The efficiency of contrast agents is investigated involving a concept of *relaxivity,* referring to the nuclear relaxation enhancement normalized to 1 mM concentration of the magnetic species. At not too high concentration of the paramagnetic species, the enhancement is proportional to that concentration. Measurements of the relaxation enhancement or relaxivity over a broad range of magnetic fields are referred to as *relaxometry,* and the resulting curve is denoted as a nu*clear magnetic relaxation dispersion* (NMRD) profile. On the experimental side, the NMRD profiles are usually measured by the field-cycling technique, where the magnetic field is rapidly switched between different values. The measured relaxivity values for Gd and Mn based contrast agents are usually in the range of 20-50 1/mM*s (D. Kruk, T. Nilsson, J. Kowalewski, Phys. Chem. Chem. Phys., 3, 4907-4917, (2001), D. Kruk, J. Kowalewski, J. Biol. Inorg. Chem., 8 (5), 512-518, (2003) ).

The publication Robert N. Muller, Luce Vander Elst, Sophie Laurent, "Spin transition molecular materials: Intelligent contrast agents for magnetic resonance imaging", J. Am. Chem. Soc., 2003, 125 (27), 8405-8407 discloses particles of Fe(II)/triazole/aminotriazole exhibiting spin transition as potential contrast agents for MRI.

The publication Chandrasekar Rajadurai, Frank Schramm, Susan Brink, Olaf Fuhr, Robert Kruk, Mohammed Ghafari, Mario Ruben, -Spin Transition in a Chainlike Supramolecular Iron (II) Complex", Inorg. Chem. (communication) , (2006), 45, 10019-10021 teaches the synthesis, structure and characterization of the spin transfer complex [Fe^{II}-(**L**) ₂H] (ClO₄) ₃. MeOH [L = 4'-(4"'-pyridyl)-1,2':6'1" -bis-(pyrazolyl)pyridine] (complex 1). The publication also describes the reversible, thermally driven spin transition at 286 K with a hysteresis loop of ca. 2 K of complex 1.

The publication of Chandrasekar Rajadurai, Olaf Fuhr, Robert Kruk, Mohammed Ghafari, Horst Hahn and Mario Ruben, "Above room temperature spin transition in a metallo-supramolecular coordination oligomer/polymer", Chem. Commun., 2007, 2636-2638 gives more information about the spin transition and other physical properties of the complex 1.

It is an object of the present invention to propose agents which can be used as a contrast agent in magnetic resonance imaging. In particular it is an object of the invention to propose an advantageous use of the above mentioned spin crossover complexes.

According to the invention the use of a spin crossover complex is proposed as a contrast agent in magnetic resonance imaging of human or animal bodies having the general formula

[M^{x+}(L_{y})H_{w}]AN)_{z}

where
M is a positively charged metal ion,
L is 4'-(4"'- pyrydyl)-1,2': 6'1"- bispyrazolylpyridine)
AN is a negatively charged inorganic anion,
w means zero, one or two
x means two or three,
y means two and
z means two, three or four

Complexes with the general formula [M^{x+} (L_{y}) H_{w}]AN)₂ with Fe²⁺ or Fe³⁺ or Co²⁺ for the metal ion M^{x+} are preferred. Especially preferred are complexes having the general formula (M^{x+}(L_{y})H_{w}]AN)_{z} with BF₄⁻ or Cl⁻ or SO₄²⁻ or ClO₄⁻ for the inorganic anion AN and with L = (4'-(4"'-pyridyl)-1,2': 6'1"-bispyrazolylpyridine) and y = 2.

Most useful are complexes with the following structure:

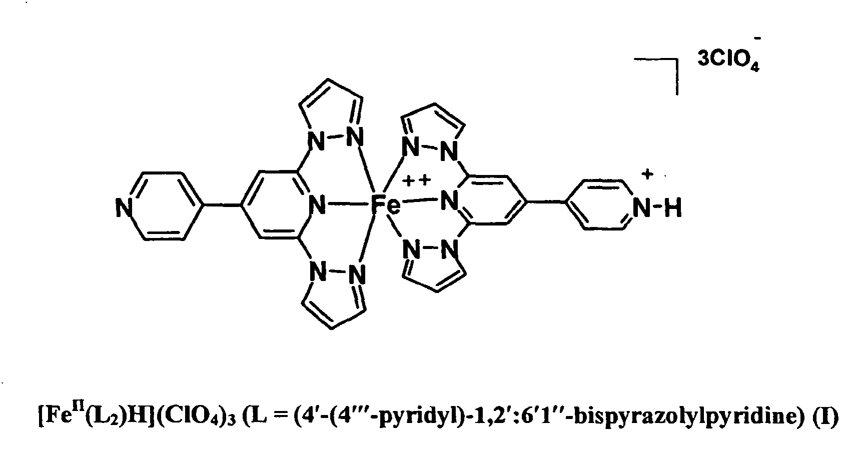

The complexes according to the invention are applied in the usual way as contrast agents, e.g. orally or intravenously. Of course, not poisonous anions are used.

In the following the invention is explained in more detail:

The above mentioned Iron(II) containing spin crossover complex **[Fe^{II} (L₂) H_{w}] (ClO₄)₃ · MeOH** (I) (w = 1) was used to test the NMRD relaxation profile of the compound with different magnetic field. In this experiment, the NMRD relaxation profiles of the compound have been collected for the discussed material.

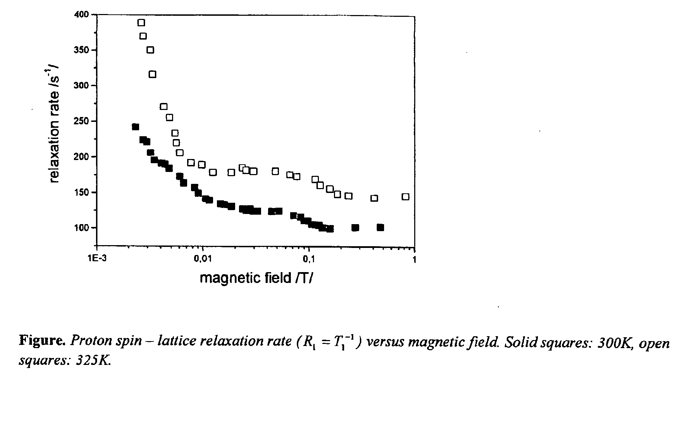

The diagram according to the figure was obtained.

One can conclude from this experiment that:
1. The proton relaxation is very efficient (taking into account comparable distances between the electron spin and proton spin in this case and in the case of the mentioned paramagnetic contrast agents)
2. One observes very significant difference between the proton relaxation rates in a guide narrow temperature range. This effect is due to the thermally driven spin transition of Fe. There is a mixture of HS (high spin) and LS (low spin) states of Fe at 300K. The relaxation enhancement is caused by the presence of the magnetic moment associated with HS Fe. Upon heating the HS fraction has further increased leading to a more efficient proton relaxation. The relaxation enhancement is very sensitive for the fraction of HS Fe.
3. We found better relaxation rates at higher temperatures (see figure, open squares), which are useful to discriminate warmer regions of the human or animal bodies, such as tumor tissue.

## Claims

1. The use of a spin crossover complex with the general formula
[M^{x+} (L₂) H_{w}] AN)_{z}
where
M is a positively charged metal ion,
L is (4'-(4'''-pyridyl)-1,2':6'1''-bispyrazolylpyridine),
AN is a negatively charged inorganic anion,
w means zero or one or two
x means two or three, and
z means two, three or four
as a contrast agent in magnetic resonance imaging of human or animal bodies.

2. The use according to claim 1 with a complex with the general formula [M^{x+}(L₂) H_{w}]AN)_{z} with Fe²⁺ or Fe³⁺ or Co²⁺ for the metal ion M^{x+}.

3. The use according to one of the claims 1 or 2 with a complex with the general formula [M^{x+}(L₂)H_{w}]AN)_{z} with BF₄⁻ or Cl⁻ or SO₄²- or ClO₄⁻ for the inorganic anion AN.

## Patentansprüche

1. Die Verwendung von Spin-Crossover Komplexen der allgemeinen Formel [M^{x+} (L₂) H_{w}]AN)_{z}, wobei
M ein positiv geladenes Metallion ist,
L (4'-(4'''-pyridyl)-1,2':6'1"-bispyrazolylpyridine) ist,
AN ein negative geladenes anorganisches Anion ist,
w bedeutet null, eins oder zwei,
x bedeutet zwei oder drei, und
z bedeutet zwei, drei oder vier,
als Kontrastmittel in der Magnetresonanztomographie von menschlichen oder tierischen Körpern.

2. Die Verwendung von Komplexen der allgemeinen Formel [M^{x+}(L₂)H_{w}]AN)_{z} nach Anspruch 1, wobei das Metallion M^{x+} aus der Gruppe Fe²⁺, Fe³⁺ oder Co²⁺ ausgewählt ist.

3. Die Verwendung von Komplexen der allgemeinen Formel [M^{x+}(L₂)H_{w}]AN)_{z} nach einem der Ansprüche 1 oder 2, wobei das anorganische Ion AN aus der Gruppe BF₄⁻, Cl⁻, SO₄²⁻ oder ClO₄⁻ ausgewählt ist.

## Revendications

1. Utilisation d'un complexe de transition de spin de la formule générale [M^{x+} (L₂) H_{w}]AN)_{z}, où
M est un ion métallique chargé positivement,
L est (4'-(4"'-pyridyl)-1,2':6'1"-bispyrazolylpyridine), AN est un anion inorganique chargé négativement,
w signifie zéro, un ou deux,
x signifie deux ou trois et
z signifie deux, trois ou quatre,
comme agent de contraste en imagerie par résonance magnétique du corps humain ou animal.

2. Utilisation d'un complexe de la formule générale [M^{x+}(L₂) H_{w}]AN)_{z} selon la revendication 1, où l'ion métallique M^{X+} est Fe²⁺, Fe³⁺ ou Co²⁺.

3. Utilisation d'un complexe de la formule générale [M^{x+}(L₂)H_{w}]AN)_{z} selon la revendication 1 ou 2, où l'anion inorganique AN est BF₄⁻, Cl⁻, SO₄²⁻, ClO₄⁻.
